# EUROPEAN PATENT APPLICATION

(11) **EP 4 729 096 A1**
(43) Date of publication of application: **22.04.2026**
(21) Application number: 24306694.1
(22) Date of filing: 15.10.2024
(51) Int. Cl.: A61M 5/32, A61M 5/31

(54) **NEEDLE COVER FOR MEDICAL INJECTION DEVICE**

(71) Applicant: Becton Dickinson France, 38800 Le Pont-de-Claix (FR)
(72) Inventor: PERROUD, Corentin, 38500 Voiron (FR); VONIEZ, Jimmy, 38000 GRENOBLE (FR); TOURNAIRE, Maxime, 38100 Grenoble (FR); TACHEGOUSTE, Nora, 38000 Grenoble (FR); SINGER, Julien, 38420 DOMENE (FR)
(74) Representative: Germain Maureau

(57) **Abstract**

A needle cover for protecting a needle mounted on a tip of a medical injection device, wherein the tip extends from a distal end of the medical injection device, including an inner shield extending along a longitudinal axis, and an outer shield surrounding at least partially the inner shield, and operatively connected to said inner shield. The outer shield may further include an extension strip provided on a proximal portion of the outer shield and extends in a distal direction to a position held between the inner shield and the tip.

## Description

### BACKGROUND OF THE INVENTION

### Field of the Invention

The present disclosure relates generally to medical injection devices for delivery of a fluid or liquid medicament. More particularly, the present disclosure relates to a needle cover adapted to be mounted on a tip of a medical injection device for covering a needle attached thereon. The disclosure also relates to a medical assembly including a medical injection device and a needle cover for enclosing the needle of the medical injection device.

### Description of Related Art

Medical injection devices such as syringes are well known in the art. These devices typically include a container for containing a medical composition such as a liquid medicament. Said container usually includes an end piece in a form of a longitudinal tip defining a fluid path through which the medical solution is expelled. A needle may be attached to the tip in order to prick the patient's skin and to perform the injection of the medical composition.

In order to maintain sterility prior to use and to reduce the risk of incurring an accidental needle-stick, protection of the needle is important. Thus, a needle cover may be mounted on the tip of the barrel so as to enclose the needle. This renders the needle physically inaccessible by the persons around the device. The needle cover may include an inner shield, in a material with elastomeric properties, and may further include an outer shield, in rigid plastic, surrounding the inner shield. In some examples, the inner needle shield may be made of a rubber material. The inner needle shield ensures the sealing of the medical injection device. To that purpose, the inner needle shield includes a sealing portion that sealingly contacts the outer surface of the syringe's tip to provide a tight seal. The inner needle shield prevents any contamination of the medical composition from the outside environment, thereby assuring the container closure integrity. The inner needle shield further prevents any leakage of composition from the outlet of the needle to the external environment. To that purpose, the needle is preferably pricked in the inner needle shield. The rigid outer shield may be made of a rigid or semi-rigid material that surrounds the inner needle shield.

The inner needle shield creates an interference force with the tip of the medical injection device to ensure a full closure of the liquid within the medical injection device. In some examples, the closure integrity of the opening of the medical injection device is created by a radial compression of the inner needle shield on the tip of the medical injection device. The radial compression on the tip of the medical injection device is increased below the tip of the medical injection device using the rigid outer needle shield to avoid any unintentional dislodgement of the needle cover off of the medical injection device. Many of these features of the needle cover also affect the amount of force needed to remove the needle cover from the medical injection device.

When designing a needle cover there are several features to take into consideration that affect the performance level of the needle cover. In particular, among these features are container closure integrity at the tip of the medical injection device, the unintentional dislodgement of the needle cover from the medical injection device, and the ability to pull the needle cover off of the medical injection device using a reasonable amount of force. These features of the needle cover often determine three different characteristics of the needle cover, which often makes it difficult to adjust a performance level for an individual feature without affecting the performance level of the remaining features.

One significant factor that contributes to a high pull-out force for a needle cover is an alignment between an internal breaking point for an inner shield and an opening on an outer shield that creates a high compression on a tip or an inner shield of the medical injection device when a tip bump on the tip has passed the breaking point of the inner shield.

### SUMMARY OF THE INVENTION

In view of the problems identified above, there is a current need for a needle cover that reduces the amount of force needed to remove the needle cover from the medical injection device.

According to one non-limiting embodiment or aspect, a needle cover for protecting a tip and a needle mounted on the tip of a medical injection device, wherein the tip extends from a distal end of the medical injection device, the needle cover, includes an inner shield extending along a longitudinal axis and comprising an outer shoulder, and an outer shield surrounding at least partially the inner shield, and operatively connected to said inner shield, the needle cover being characterized in that: wherein a breaking point of an inner surface of the inner shield is positioned on the inner shield at a vertical location above the outer shoulder or a vertical location below the outer shoulder.

According to one non-limiting embodiment or aspect, the distance between the outer shoulder and the breaking point of the inner surface of the inner shield may be no more than 0.3 mm. A distance between the outer shoulder and a breaking point of an inner surface of the inner shield may be in the range of 0 mm to 0.3 mm. The breaking point may be positioned on the inner shield at only the vertical location below the outer shoulder. The breaking point may be positioned at only at the vertical location above the outer shoulder.

According to one non-limiting embodiment or aspect, a needle cover for protecting a tip and a needle mounted on the tip of a medical injection device, wherein the tip extends from a distal end of the medical injection device, the needle cover, may include an inner shield extending along a longitudinal axis and comprising an outer shoulder, and an outer shield surrounding at least partially the inner shield, and operatively connected to said inner shield, the needle cover being characterized in that: a breaking point established on an inner surface of the inner shield is positioned at an angle between 100 degrees and 160 degrees.

According to one non-limiting embodiment or aspect, the angle is no more than 160 degrees. The angle may be measured between a vertical surface defined on the inner surface of the inner shield and a substantially horizontal surface defined on the inner surface of the inner shield. The angle may be 100 degrees. The angle may be 130 degrees.

According to one non-limiting embodiment or aspect, a needle cover for protecting a tip and a needle mounted on the tip of a medical injection device, wherein the tip extends from a distal end of the medical injection device, the needle cover, may include an inner shield extending along a longitudinal axis and comprising an outer shoulder, and an outer shield surrounding at least partially the inner shield, and operatively connected to said inner shield, the needle cover being characterized in that: an inner surface of the inner shield defines at least two chamfers that are angled relative to a longitudinal axis of the inner shield.

According to one non-limiting embodiment or aspect, a first chamfer may be defined on the inner surface of the inner shield and a second chamfer may be defined on the inner surface of the inner shield. The first chamfer may be provided at a greater angle relative to the longitudinal axis of the inner shield than the second chamfer. The first chamfer may be measured at an angle of 70 degrees from the longitudinal axis of the inner shield.

The invention is further disclosed in the following clauses:
Clause 1: A needle cover for protecting a tip and a needle mounted on the tip of a medical injection device, wherein the tip extends from a distal end of the medical injection device, the needle cover, comprising: an inner shield extending along a longitudinal axis and comprising an outer shoulder, and an outer shield surrounding at least partially the inner shield, and operatively connected to said inner shield, the needle cover being characterized in that: wherein a breaking point of an inner surface of the inner shield is positioned on the inner shield at a vertical location above the outer shoulder or a vertical location below the outer shoulder.
Clause 2: The needle cover of Clause 1, wherein the distance between the outer shoulder and the breaking point of the inner surface of the inner shield is no more than 0.3 mm.
Clause 3: The needle cover of Clause 1 or Clause 2, a distance between the outer shoulder and a breaking point of an inner surface of the inner shield is in the range of 0 mm to 0.3 mm.
Clause 4: The needle cover of Clause 1 or Clause 2, wherein the breaking point is positioned on the inner shield at only the vertical location below the outer shoulder.
Clause 5: The needle cover of Clause 1 or Clause 2, wherein the breaking point is positioned at only at the vertical location above the outer shoulder.
Clause 6: A needle cover for protecting a tip and a needle mounted on the tip of a medical injection device, wherein the tip extends from a distal end of the medical injection device, the needle cover, comprising: an inner shield extending along a longitudinal axis and comprising an outer shoulder, and an outer shield surrounding at least partially the inner shield, and operatively connected to said inner shield, the needle cover being characterized in that: a breaking point established on an inner surface of the inner shield is positioned at an angle between 100 degrees and 160 degrees.
Clause 7: The needle cover of Clause 6, wherein the angle is no more than 160 degrees.
Clause 8: The needle cover of Clause 6 or Clause 7, wherein the angle is measured between a vertical surface defined on the inner surface of the inner shield and a substantially horizontal surface defined on the inner surface of the inner shield.
Clause 9: The needle cover of any of Clauses 6-8, wherein the angle is 100 degrees.
Clause 10: The needle cover of any of Clauses 6-8, wherein the angle is 130 degrees.
Clause 11: A needle cover for protecting a tip and a needle mounted on the tip of a medical injection device, wherein the tip extends from a distal end of the medical injection device, the needle cover, comprising: an inner shield extending along a longitudinal axis and comprising an outer shoulder, and an outer shield surrounding at least partially the inner shield, and operatively connected to said inner shield, the needle cover being characterized in that: an inner surface of the inner shield defines at least two chamfers that are angled relative to a longitudinal axis of the inner shield.
Clause 12: The needle cover of Clause 11, wherein a first chamfer is defined on the inner surface of the inner shield and a second chamfer is defined on the inner surface of the inner shield.
Clause 13: The needle cover of Clause 12, wherein the first chamfer is provided at a greater angle relative to the longitudinal axis of the inner shield than the second chamfer.
Clause 14: The needle cover of Clause 12, wherein the first chamfer is measured at an angle of 70 degrees from the longitudinal axis of the inner shield.

### BRIEF DESCRIPTION OF THE DRAWINGS

FIG. 1 is a cross-sectional view of a needle cover according to at least one non-limiting aspect or embodiment of the present disclosure;
FIG. 2 is a cross-sectional view of another needle cover according to at least one non-limiting aspect or embodiment of the present disclosure;
FIG. 3 is a cross-sectional view of another needle cover according to at least one non-limiting aspect or embodiment of the present disclosure;
FIG. 4 is a cross-sectional view of another needle cover according to at least one non-limiting aspect or embodiment of the present disclosure; and
FIG. 5 is a cross-sectional view of another needle cover according to at least one non-limiting aspect or embodiment of the present disclosure.
FIG. 6 is a side view of a medical injection device covered by a needle cover according to one non-limiting aspect or embodiment of the present disclosure.
FI. 7 is a cross-sectional side view of the medical injection device of FIG. 6.

### DESCRIPTION OF THE DISCLOSURE

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the following discussion, "distal" refers to a direction generally toward an end of a medical injection device adapted for contact with a patient's skin, and "proximal" refers to the opposite direction of distal, i.e., away from the end of a medical injection device. In other words, the "distal direction" is to be understood as meaning the direction of injection. The distal direction corresponds to the travel direction of the plunger during the injection, the medical composition contained initially in the barrel being expelled from the latter. The "proximal direction" is to be understood as meaning the opposite direction to said direction of injection. For purposes of this disclosure, the above-mentioned references are used in the description of the components of a medical injection device in accordance with the present disclosure.

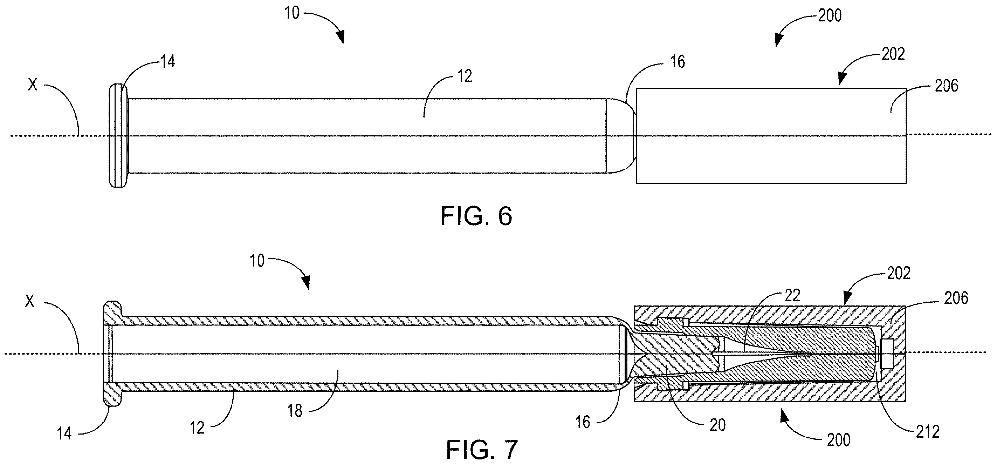

Referring to FIGS. 6 and 7, the medical injection device 10 may include a barrel 12 extending along a longitudinal axis X from a proximal face 14 to a distal face 16, defining a reservoir 18 for a medical composition such as a liquid medicament. The medical injection device 10 may also include a stopper (not shown), and a plunger rod (not shown) translationally movable inside the barrel 12 from a proximal position to a distal position for injecting the composition.

The medical injection device 10 further includes a distal tip 20 extending along the longitudinal axis X from the distal face 16 of the barrel 12. The distal tip 20 may be at least partially hollow so as to form a fluid path in fluidic communication with the barrel 12. A needle 22 may be attached to the distal tip 20 of the medical injection device 10 and may be in fluid communication with the fluid path. It is noted herein that the distal face 16 of the barrel 12 is proximate a shoulder of the medical injection device 10.

The medical injection device 10 is preferably made of glass, and more preferably is a glass syringe. Such glass syringes are largely used in hospital environments and readily sterilizable. In other examples of the present disclosure, the medical injection device 10 may be made of a medical grade plastic. The medical injection device 10 is preferably a prefilled or a pre-fillable syringe. The medical injection device 10 is more preferably a syringe with the needle 22.

With continued reference to FIGS. 6 and 7, the medical injection device 10 may further include a needle cover 200. The needle cover 200 may shield and cover the needle 22. The needle cover 200 may include an outer shield 202 and an inner shield 204 that covers the needle 22. In one embedment, the outer shield 202 covers at least a portion of the inner shield 204. In another embodiment, the outer shield 202 covers the entire inner shield 204.

The outer shield 202 in accordance with an example of the present disclosure is described in greater detail. The outer shield 202 is preferably made in a rigid material, such as rigid plastic. The outer shield 202 may be made of a medical grade plastic. The rigid material confers rigidity to the outer shield 202, which allows said outer shield 202 to better protect the needle cover 200 from shocks. The structural integrity of the needle cover 200 is thereby improved.

The outer shield 202 includes a body 206 having a proximal end and a distal end. The body 206 extends along a longitudinal axis. The longitudinal axis coincides with the longitudinal axis X when the needle cover 200 is mounted on the tip 20 of the medical injection device 10.

In one example, the body 206 may be substantially cylindrical in shape. The body 206 may define an inner cavity 212 that at least partially receives the inner shield 204 of the needle cover 200.

With continued reference to FIGS. 6 and 7, the inner shield 204 according to one example of the present disclosure is described. The inner shield 204 may extend along a longitudinal axis. The longitudinal axis may coincide with the longitudinal axis X when the inner shield 204 is at least partly enclosed in the outer shield 202. The inner shield 204 preferably has a cylindrical shape and a circular cross section.

The inner shield 204 is preferably made in a material with elastomeric properties. The inner shield 204 may be made of a soft material including an elastomer or a thermoplastic elastomer. In this way, at least a portion of the inner shield 204 may slightly deform when connecting the inner shield 204 to the medical injection device 10 so as to match the shape of the tip 20. Meanwhile, the distal part of the needle 22 may penetrate the inner shield 204. This further reduces the risk of leakage of the medical composition via the needle 22 to the external environment. The material with elastomeric properties is preferably a thermoplastic elastomer, an elastomer, or a rubber. Preferably, the material with elastomeric properties is sterilizable.

In one embodiment, the outer shield 202 surrounds at least partially the inner shield 204. The outer shield 202 is fixed to the inner shield 204. The inner shield 204 and the outer shield 202 may be fixed together by a snap-fit connection or a friction fit, among other connection methods. When the needle cover 200 is mounted on the medical injection device 10, the inner shield 204 encloses at least a portion of the tip 20 of the barrel 12. The needle cover 200 is thus tightly and sealingly connected to the tip 20. In one embodiment, the inner shield 204 creates a sufficiently tight seal with the tip 20 of the medical injection device 10 to ensure the sterility and fluid tight seal between the tip 20 of the medical injection device 10 and the inner shield 204.

In order to mount the needle cover 200 on the medical injection device 10, the outer shield 202 is first placed over the inner shield 204, and then the entire needle cover 200 is mounted on the medical injection device 10. After the needle cover 200 has been positioned on the medical injection device 10, the needle cover 200 can be moved in a distal direction to remove it from the medical injection device 10. As a pulling force is applied to the outer shield 202 by a user, the outer shield 202 is moved in the distal direction along with the inner shield 204. As the outer shield 202 is moved in the distal direction, a proximal end of outer shield 202 will begin to contact and abut a proximal portion of the inner shield 204. The proximal portion of the inner shield 204 is pushed in the distal direction. This compression of the inner shield 204 creates a mechanical deformation of the inner shield 204, thereby constricting the inner shield 204 on the outer shield 202 in the radial direction.

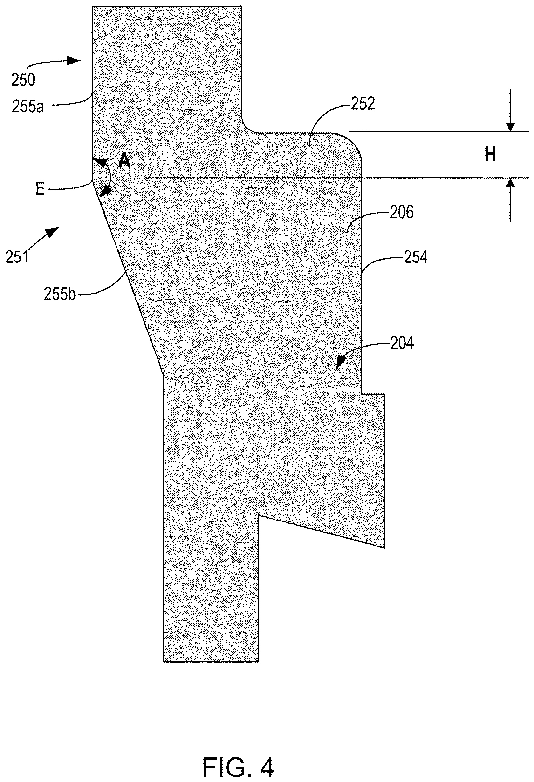

With reference to FIG. 4, according to one non-limiting embodiment or aspect of the present disclosure, the needle cover 200 may include features that are provided to avoid residual critical strains that create high pull-out forces for the needle cover 200. These features of the needle cover 200 are provided to reduce or eliminate interference between the outer shield 202 and the inner shield 204 when the needle cover 200 is at a maximal load for the pull-out force. The inner shield 204 includes an inner surface 250 that is configured and positioned in the inner shield 204 to contact the tip 20 of the medical injection device 10. This inner surface 250 of the inner shield 204 ensures cap integrity of the needle cover 200. One end 251 of the inner surface 250 is delimited by a breaking point E leading to a thickness reduction for the inner shield 204 to release the tip 20 of the medical injection device 10 from any contact. In one non-limiting aspect or embodiment of the present disclosure, the breaking point E may be understood to be a delimitation point for the end of the contact between the tip 20 and the inner surface 250.

With continued reference to FIG. 4, the inner shield 204 also includes a shoulder 252 defined by an outer surface 254 of the inner shield 204. The needle cover 200 is assembled due to the shoulder 252 of the inner shield 204 being maintained in the outer shield 202. In one aspect, openings of the outer shield 202 and the shoulder 252 of the inner shield 204 overlap with the breaking point E in the same axial plane. This established overlap can be a significant factor in increasing the amount of pull-out force when the tip 20 of the medical injection device 10 is passing the breaking point E when the needle cover 200 is being removed from the medical injection device 10. However, by shifting or moving the breaking point E away from the openings 222 of the outer shield by a distance H, the required pull-out force is reduced. This feature can be achieved by adapting the needle cover 200 in several different manners. By adapting the needle cover 200 in any of these manners, the compressed material volume of the inner shield 204 is reduced to reduce the pull-out force needed to remove the needle cover 200. Further, an existing gap between the outer shield 202 and the inner shield 204 allows for a deformation of the inner shield 204 without compression at the breaking point E. Therefore, any strains applied during the removal of the needle cover 200 are more evenly distributed along the longitudinal axis of the inner shield 204 and any compression strains applied on the tip 20 of the medical injection device 10 are reduced.

In one aspect, a position of the shoulder 252 on the inner shield 204 and the openings 222 of the outer shield 202 on the needle cover 200 may be modified. In another aspect, a position of the breaking point E of the inner shield 204 may be modified. In another aspect, a depth of the needle cover 200 may be reduced to avoid the tip 20 from bumping the inner surface 250 of the inner shield 204 when the needle cover 200 is removed from the tip 20. One goal of the present disclosure is to reduce the inner shield material that might be compressed in the outer shield window. This can be particularly feasible when changing the position of the openings or of the breaking point E. Another skilled way would be to adapt the angle for a frozen design of openings and the breaking point E so that the aforesaid material quantity might be slightly reduced.

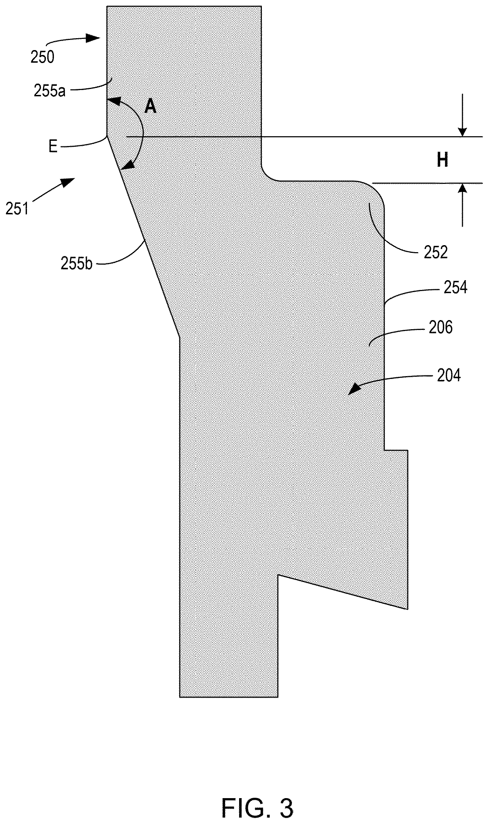

With reference to FIGS. 3 and 4, according to one or more non-limiting aspects or embodiments of the present disclosure, a height H is measured between the breaking point E of the inner shield 204 and the upper surface of the outer shoulder 252 of the inner shield 204. The vertical distance measured for this height H may be adjusted to reduce the amount of pull-out force needed to remove the needle cover 200. In particular, in some examples, this height H can measure from 0 mm to 0.3 mm. As illustrated in FIG. 3, the height H can be measured from the upper surface of the outer shoulder 252 to a breaking point E positioned above the upper surface of the outer shoulder 252 when the medical injection device 10 is positioned in an upright position such that the tip 20 of the medical injection device 10 is positioned vertically above the barrel 12. As illustrated in FIG. 4, the height H can also be measured from the upper surface of the outer shoulder 252 to a breaking point E positioned below the upper surface of the outer shoulder 252 when the medical injection device 10 is positioned in an upright position such that the tip 20 of the medical injection device 10 is positioned vertically above the barrel 12. By establishing the height H with the inner shield 204, the unexpected result of reducing the pull-out force for the needle cover 200 was achieved. This measured height H is critical to achieving the reduced pull-out force since this measured height H eliminates or reduces the overlap between the breaking point E and the openings 222 of the outer shield 202.

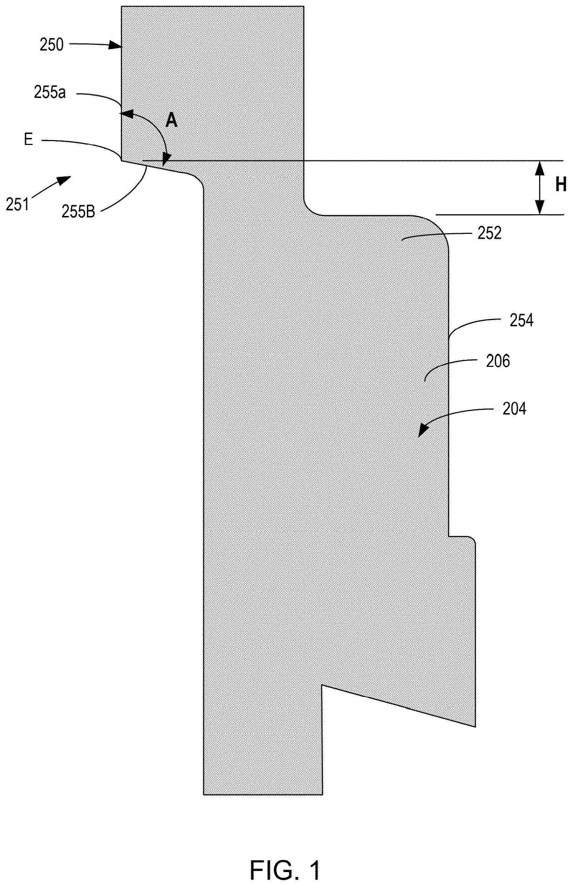

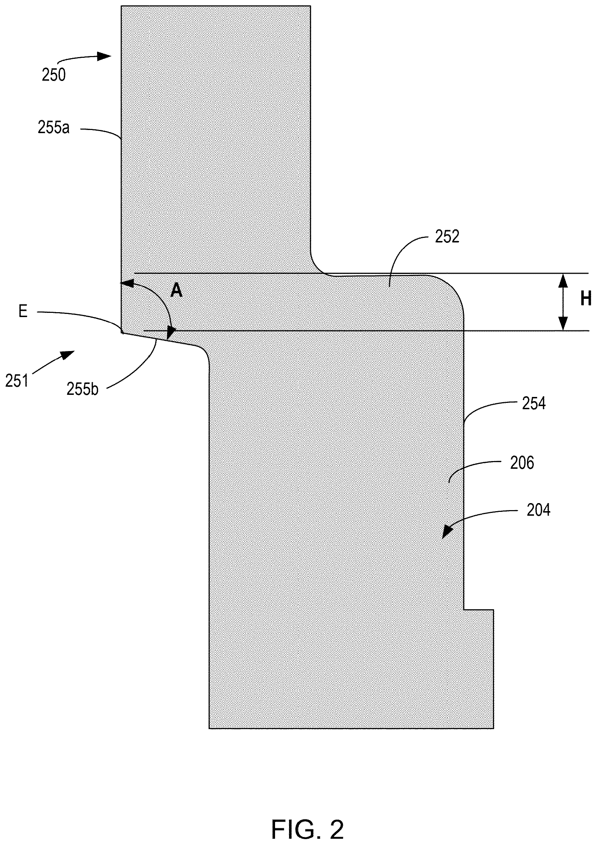

With continued to reference to FIGS. 1-4, according to one or more non-limiting aspects or embodiments of the present disclosure, an angle A established on the inner surface 250 of the inner shield 204 may be adjusted to reduce the pull-out force. The angle A may be measured between a first vertical surface 255a and a surface 255b that extends substantially perpendicular to the first vertical surface 255a, even though this surface 255b may be slightly angled relative to a vertical plane. Each of the first vertical surface 255a and the surface 255b form part of the inner surface 250 of the inner shield 204. In some examples of the present disclosure, the angle A may measure between 100 degrees and 160 degrees. In particular, the angle A may measure either 100 degrees, 130 degrees, or 160 degrees. It is to be understood that this angle A may also be any alternative angle in the range of 100 degrees to 160 degrees. As illustrated in FIG. 1, the angle A can be measured from the upper surface of the outer shoulder 252 to a vertical surface 255a above the upper surface of the outer shoulder 252 when the medical injection device 10 is positioned in an upright position such that the tip 20 of the medical injection device 10 is positioned vertically above the barrel 12. The angle A can also be measured from the upper surface of the outer shoulder 252 to a vertical surface 255a positioned below the upper surface of the outer shoulder 252 when the medical injection device 10 is positioned in an upright position such that the tip 20 of the medical injection device 10 is positioned vertically above the barrel 12. Similar to the height H, by establishing the angle A with the inner shield 204, the unexpected result of reducing the pull-out force for the needle cover 200 was achieved. This measured angle A is critical to achieving the reduced pull-out force since this measured angle A eliminates or reduces the overlap between the breaking point E and the openings 222 of the outer shield 202. It is also to be understood that the inner shield 204 may be modified by adjusting both the height H and the angle A of the inner shield in combination to achieve the reduced pull-out force.

By adjusting the height H of the inner shield 204, it has been identified that the higher the value of the height H, the more flexible the area of the inner shield 204 between the barrel 12 and the outer shield 202. By increasing the flexibility of this area of the inner shield 204, the greater the reduction in the pull-out force for the needle over 200. Provided below is a chart identifying the reduced pull-out forces achieved by modified the height H and the angle A of the inner shield 204. It is to be noted that a negative height H measurement is to be understood that this height H is measured at a point below the outer shoulder 252 of the inner shield 202. In one non-limiting embodiment or aspect of the present disclosure, angle A should be defined so that the inner shield material that might be compressed in the outer shield window is not greater than the material quantity when H=-0.3mm & angle =130°C.

| Height (H) | Angle (A) | Pull-Out Force |
|---|---|---|
| -0.3 mm | 100 degrees | 9.5 N |
| -0.3 mm | 130 degrees | 11.5 N |
| -0.3 mm | 160 degrees | 12.5 N |
| 0 mm | 100 degrees | 7.5 N |
| 0 mm | 130 degrees | 8.5 N |
| 0 mm | 160 degrees | 9.5 N |
| 0.3 mm | 100 degrees | 6.0 N |
| 0.3 mm | 130 degrees | 7.0 N |
| 0.3 mm | 160 degrees | 8.0 N |

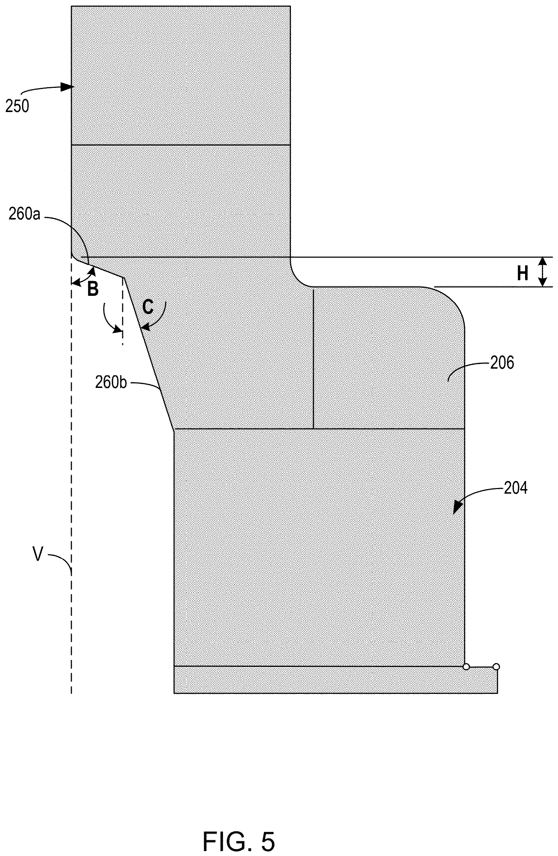

With reference to FIG. 5, according to another non-limiting aspect or embodiment of the present disclosure, the inner surface 250 of the inner shield 204 can be modified in a different manner to reduce the pull-out force of the needle cover 200. In particular the inner surface 250 may define two chamfers 260a, 260b to assist in reducing the pull-out force. A first chamfer 260a may be positioned at an angle B relative to the inner surface 250 of the inner shield 204. In one example, angle B may be 70 degrees. The angle B may be measured between a longitudinal axis V of the inner shield 204 and the surface of the first chamfer 260a. A second chamfer 260b may be positioned at an angle C relative to the inner surface 250 of the inner shield 204. The angle C may be measured between the longitudinal axis V of the inner shield 204 and the surface of the second chamfer 260b. This modification of the inner surface 250 of the inner shield 204 will also assist in reducing the pull-out force for the needle cover 200.

While this disclosure has been described as having exemplary designs, the present disclosure can be further modified within the spirit and scope of this disclosure. This application is therefore intended to cover any variations, uses, or adaptations of the disclosure using its general principles. Further, this application is intended to cover such departures from the present disclosure as come within known or customary practice in the art to which this disclosure pertains and which fall within the limits of the appended claims.

## Claims

1. A needle cover for protecting a tip and a needle mounted on the tip of a medical injection device, wherein the tip extends from a distal end of the medical injection device, the needle cover, comprising:
an inner shield extending along a longitudinal axis and comprising an outer shoulder, and
an outer shield surrounding at least partially the inner shield, and operatively connected to said inner shield,
the needle cover being **characterized in that**:
wherein a breaking point of an inner surface of the inner shield is positioned on the inner shield at a vertical location above the outer shoulder or a vertical location below the outer shoulder.

2. The needle cover of claim 1, wherein the distance between the outer shoulder and the breaking point of the inner surface of the inner shield is no more than 0.3 mm.

3. The needle cover of claim 1 or claim 2, a distance between the outer shoulder and a breaking point of an inner surface of the inner shield is in the range of 0 mm to 0.3 mm.

4. The needle cover of claim 1 or claim 2, wherein the breaking point is positioned on the inner shield at only the vertical location below the outer shoulder.

5. The needle cover of claim 1 or claim 2, wherein the breaking point is positioned at only at the vertical location above the outer shoulder.

6. A needle cover for protecting a tip and a needle mounted on the tip of a medical injection device, wherein the tip extends from a distal end of the medical injection device, the needle cover, comprising:
an inner shield extending along a longitudinal axis and comprising an outer shoulder, and
an outer shield surrounding at least partially the inner shield, and operatively connected to said inner shield,
the needle cover being **characterized in that**:
a breaking point established on an inner surface of the inner shield is positioned at an angle between 100 degrees and 160 degrees.

7. The needle cover of claim 6, wherein the angle is no more than 160 degrees.

8. The needle cover of claim 6 or claim 7, wherein the angle is measured between a vertical surface defined on the inner surface of the inner shield and a substantially horizontal surface defined on the inner surface of the inner shield.

9. The needle cover of any of claims 6-8, wherein the angle is 100 degrees.

10. The needle cover of any of claims 6-8, wherein the angle is 130 degrees.

11. A needle cover for protecting a tip and a needle mounted on the tip of a medical injection device, wherein the tip extends from a distal end of the medical injection device, the needle cover, comprising:
an inner shield extending along a longitudinal axis and comprising an outer shoulder, and
an outer shield surrounding at least partially the inner shield, and operatively connected to said inner shield,
the needle cover being **characterized in that**:
an inner surface of the inner shield defines at least two chamfers that are angled relative to a longitudinal axis of the inner shield.

12. The needle cover of claim 11, wherein a first chamfer is defined on the inner surface of the inner shield and a second chamfer is defined on the inner surface of the inner shield.

13. The needle cover of claim 12, wherein the first chamfer is provided at a greater angle relative to the longitudinal axis of the inner shield than the second chamfer.

14. The needle cover of claim 12, wherein the first chamfer is measured at an angle of 70 degrees from the longitudinal axis of the inner shield.
